# EUROPEAN PATENT APPLICATION

(11) **EP 0 616 836 A2**
(43) Date of publication of application: **28.09.1994**
(21) Application number: 94103376.3
(22) Date of filing: 05.03.1994
(51) Int. Cl.: B01D 61/00, G01N 15/00

(54) **Separation system including apparatus and method**

(30) Priority: 23.03.1993 US 36012
(71) Applicant: Becton Dickinson and Company, Franklin Lakes, New Jersey 07417-1880 (US)
(72) Inventor: Uffenheimer, Kenneth Frank, Los Gatos, California 95030 (US)
(74) Representative: Selting, Günther, Dipl.-Ing.

(57) **Abstract**

A system includes an apparatus and a method for separating objects such as cells, particles and the like from at least a portion of the fluid in which they are suspended. The apparatus includes a source (12) of fluid (13) with the objects suspended therein, a collection member (14) for retaining at least a portion of the fluid, a pump (52) for circulating the fluid along a flow path through a filter assembly (16) and back to the collection member, and a valve (58) for maintaining the level of the fluid in the collection member (14) within a range around the low level sensor (20). The method of separating such objects includes providing the fluid with the objects suspended therein, circulating the fluid (13) through the filtration assembly (16), drawing off a portion of the fluid with the objects removed therefrom and monitoring the level of the fluid in the collection member (14). In this way, the apparatus and method of the present system are suitable for use to separate objects such as cells, particles and the like for concentration with an acceptable degree of recoverability and viability.

## Description

### FIELD OF THE INVENTION

The present invention generally relates to a system for separating one material from another, and more specifically relates to an apparatus and method for separating cells, particles or like objects from at least a portion of a fluid in which such objects are suspended, with the objects, for example, having been previously sorted or otherwise separated by a flow cytometer. In this way, the apparatus and method may be used, for example, to concentrate the objects.

### BACKGROUND OF THE INVENTION

Flow cytometry, the measurement and/or separation of objects such as cells and other particles in a moving liquid stream, is well established as a valuable analysis tool in research and clinical laboratories. A discussion of the various principles, techniques and apparatus behind flow cytometry is set forth in an article by John L. Haynes, entitled "Principles of Flow Cytometry", Cytometry Supplement 3:7-17 (1988), the disclosure of which is hereby incorporated by reference.

Conventional flow cytometry devices for sorting objects such as cells and particles with specific characteristics basically consist of a liquid stream forming a sheath to focus the objects as they pass through an orifice associated with the analyzing or counting capabilities of the device. Usually, the objects can be sorted at high speeds to collect tens of thousand of the objects based on a variety of chemical and physical characteristics such as size, granulation of the cytoplasm and presentation of specific antigens. Accordingly, there has been considerable interest in flow cytometry to sort objects for subsequent analysis.

In one type of sorting cytometer, after detecting the desired characteristic with an optical electronic system, the stream containing the individual objects is electrically charged shortly before being broken into droplets containing individual objects. An electrostatic field then diverts the flow of object-containing droplets into two or more streams depending on the polarity and charge of the droplets, and the divided streams are collected in separate containers as disclosed, for example, in U.S. Patent No. 4,230,558 (Fulwyler), the disclosure of which is hereby incorporated in its entirety by reference.

In another type of sorting cytometer, the stream is likewise broken into droplets for collection with the portion of the stream containing the objects of interest collected after detection by the optical electronic system. In contrast to the first type, the sorting may be accomplished by deflection of the stream as in the fluidics switching flow sorter described, for example, in U.S. Patent No. 4,175,662 (Zöld), the disclosure of which is hereby incorporated in its entirety by reference. Fluidics switching sorters have a closed fluidics system and use gas controlled by piezoelectric valves to divert the fluid stream to sort the objects.

Alternatively, the portion of the stream containing the desired objects may be collected by moving a catcher tube in the focused liquid stream at an appropriate time for intermittently receiving selected objects as described, for example, in U.S. Patent No. 5,030,002 (North, Jr.), the disclosure of which is hereby incorporated in its entirety by reference. The catcher tube sorter does not rely on deflection of the objects for sorting and therefore has the advantages of avoiding pressure pulses, minimizing damage to cells and reducing or eliminating biologically hazardous aerosols. In addition, this sorter is configured so that the catcher tube sits continuously in the edge of the fluid stream but is not aligned with the portion of the stream which contains objects until it is moved into the center of the stream to collect the desired object, i.e., cell, particle or the like. The catcher tube sorter therefore collects a relatively larger volume of fluid, i.e., relative to the amount of objects sorted, than does either the droplet type sorter or the fluidics type switching sorter, with the result that more rare the collected object, the more dilute the resulting object suspension.

Accordingly, several disadvantages and limitations have been encountered in connection with the subsequent analysis of such objects sorted by these devices as a result of the selected objects being suspended in a large volume of sheathing liquid. In an effort to minimize these disadvantages and limitations, several techniques have been proposed and utilized to concentrate or otherwise separate the objects from the larger volume of sheathing fluid. Such attempts have included subsequent concentration by filtration, centrifugation, as described in U.S. Patent Nos. 4,244,513 (Foyer et al.), 3,850,369 (Bull et al.) and 3,824,841 (Bull), and high-gradient magnetic separation ("HGMS"), as described in U.S. Patent Nos. 4,665,595 (Graham) and 4,664,796 (Graham et al.).

These attempts at concentrating the objects have presented other problems. For example, filtration has effected recoverability and viability as a result of the packing of the objects against the filter. Centrifugation is inefficient when the cell suspension is dilute, and a substantial loss of cells often results. In addition, centrifugation requires large receptacles, e.g., 50 ml tubes, which subsequently needed decanting to eliminate the excess liquid. Also, when very few cells are present in a large volume, recovery of any cells by centrifugation may be impossible or impractical. HGMS requires multiple steps to first hold or capture the objects in a matrix and a then retrieve the captured objects. Also these separation techniques have proved detrimental to the viability of the objects.

In addition, these techniques, including tangential filtration systems, have failed to address the need for a walk-away system which could be integrated into existing flow cytometers or provided as a stand-alone device to quickly recover viable objects.

Thus, there has been a need for a system for separating objects from at least a portion of the fluid in which the objects are suspended, which could be used to concentrate the objects and which would eliminate the problems and limitations associated with the prior techniques discussed above, most significant of the problems being associated with the recoverability and viability of the sorted objects. In addition, there has been a need for a system which could be integrated into existing flow cytometers or provided as a stand alone system. Also, there has been a need for such a system which would provide walk-away operation by monitoring and controlling the level of the fluid within an acceptable range.

### SUMMARY OF THE INVENTION

In contrast to the prior techniques discussed above, it has been found that a system, including an apparatus and method, particularly suited for use to separate objects such as cells, particles and the like, from at least a portion of a fluid in which they are suspended, while providing acceptable levels of recoverability and viability can be constructed in accordance with the present invention. In addition, the system of the present invention can be used either integrally or independently with existing sorting devices white providing a walk-away system to, for example, concentrate the objects.

The apparatus of the present invention for separating objects such as cells, particles and the like from at least a portion of a fluid in which they are contained or otherwise suspended includes source means for supplying a fluid with the objects to be separated suspended therein, collection means for retaining at least partially diluted objects, filtration means for filtering at least a portion of the objects from the fluid, circulating means for circulating the fluid from the collection means through the filtration means while at least a portion of the fluid may be drawn off to concentrate the objects in a remaining portion of the fluid, while permitting the objects suspended in the remaining portion of the fluid to continue to be circulated through the apparatus, and fluid level control means for monitoring and controlling the level of fluid in the collection means.

In the preferred embodiment, the fluid level control means is adapted to include the sensing means for detecting when the fluid approaches a high level point and for detecting when the fluid approaches a low level point. In addition, the fluid level control means is adapted to include a lifting means for raising and lowering the collection means, with the lifting means including a lifter valve, a source of compressed air and a cylinder connected to the lifer, with the cylinder in communication with the source of compressed air. The fluid level control means is also adapted to lower the collection means to drain off any remaining fluid from the flow path upon completion of a concentration phase. Also, the filtration means is adapted to include a filter membrane positioned along a flow path through which the portion of fluid is drawn off and adapted to include filtrate removal means for drawing off the portion of the fluid through the filter membrane. Further, the filtrate removal means includes at least one pump and a valve so that when the valve is in a first position the portion of fluid is drawn through the filter membrane. The circulating means is adapted to include a circulating pump for circulating the fluid from the collection means through the filter assembly and back to the collection means.

The method of the present invention for separating objects such as cells, particles and the like from at least a portion of a fluid in which such objects are suspended includes the steps of providing the fluid with the objects suspended therein to a collection member, circulating the fluid from the collection member along a flow path through a filtration assembly and returning at least a remaining portion of the fluid to the collection member with a higher concentration of objects suspended therein, drawing off a portion of the fluid from the filtration assembly as the fluid circulates therethrough, while permitting the objects suspended in the remaining fluid to continue to be circulated along the flow path, and monitoring and controlling the level of the fluid in the collection member.

In the preferred embodiment, the method further includes the step of passing the fluid through the filtration assembly tangentially to a first side of a filter membrane with sufficient force to prevent the objects from being lodged against the first side of the filter membrane while permitting the portion of the fluid to pass through the filter membrane and be drawn off from the remaining portion of fluid circulating along the flow path through the circulating means. The step of passing the fluid through the filtration assembly tangentially to a first side of a filter membrane includes initially directing the fluid towards the filter membrane at an acute angle. In addition, the method includes the step of providing a negative pressure difference to a second side of the filter assembly so that the portion of the fluid is drawn through the filter membrane to a holding member. The method also includes the steps of raising the collection member to further concentrate the objects in the fluid and thereafter lowering the collection member to drain off any remaining fluid from the flow path. Further, the method includes the steps of sensing whether the tube is in place, and resetting the system if the sensor is activated.

### BRIEF DESCRIPTION OF THE DRAWINGS

The various features, objects, benefits, and advantages of the present invention will become more apparent upon reading the following detailed description of the preferred embodiment along with the appended claims in conjunction with the drawings, wherein like reference numerals identify corresponding components, and:
Figure 1 is a schematic representation of the system of the present invention;
Figure 2 is a schematic, enlarged cross sectional view of the filtration assembly of the system illustrated in Figure 1;
Figures 3A and 3B are schematic representations of the sensors of the sensor assembly used in the system of the present invention;
Figures 4 and 4A are various views of the bulkhead assembly, with Figure 4 being a side view and Figure 4A being a bottom view;
Figures 5, 5A, 5B and 5C are various views of the top portion of the filtration assembly, with Figure 5 being a top view, Figures 5A and 5B being cross-sectional views taken through lines 5A-5A and 5B-5B, respectively, of Figure 5 and Figure 5C being a bottom view of the top portion;
Figures 6, 6A, 6B and 6C are various views of the bottom portion of the filtration assembly, with Figure 6 being a top view and Figures 6A, 6B and 6C being cross-sectional views taken through lines 6A-6A, 6B-6B and 6C-6C, respectively, of Figure 6;
Figures 7 and 7A are views of the clamping washer for the filtration assembly, with Figure 7 being a top view and Figure 7A being a cross-sectional view taken through line 7A-7A of Figure 7;
Figures 8A, 8B and 8C are various views of the screw of the filtration assembly, with Figure 8A being a frontal view, Figure 8B being a side view and Figure 8C being a top view;
Figures 9A, 9B and 9C are logic flow diagrams depicting the various steps for the method of the present invention, with Figure 9A depicting the steps during the separation phase of the method and Figures 9B and 9C depicting the steps during the final phase in which any remaining fluid is drained from the system; and
Figure 10 is a timing diagram depicting the operation of the various portions of the system shown in Figures 1 and 3.

### DETAILED DESCRIPTION OF THE PREFERRED EMBODIMENT

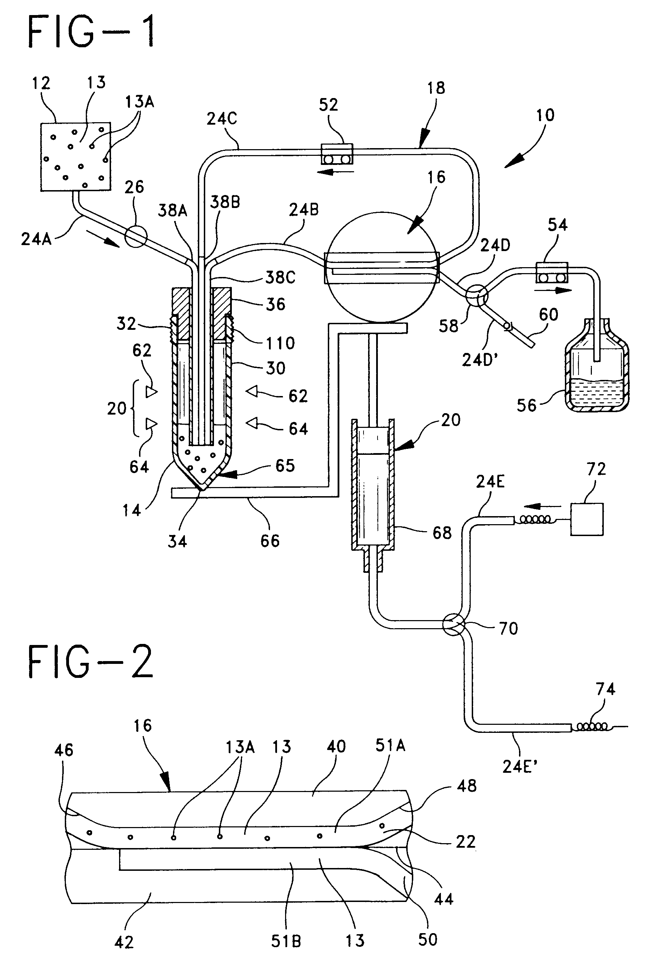

The system of the present invention is illustrated in Figures 1-10 and includes an apparatus and a method for separating objects, such as cells, particles and the like, suspended in a fluid from at least a portion of the fluid. In this way, the apparatus and method can be used to, for example, concentrate the objects. The apparatus is schematically illustrated in Figures 1 and 2 and generally designated as 10.

Referring to Figure 1, the apparatus 10 includes a source or supply 12 of fluid 13 with diluted objects 13A suspended therein, a collection member or vessel 14, a filtration assembly 16, a circulating assembly 18, and a sensor assembly 20. The fluid 13 is supplied to and circulated through the various assemblies through various lengths of tubing 24 along a flow path 22 thereof. The particular type of tubing is not essential to the present invention and may include, for example, plastic, glass or metal tubing or any combination thereof.

The particular shape or configuration of the collection member 14 is not essential to the present invention, which in the preferred embodiment is a plastic tube 30 such as for example a Falcon brand centrifugal 15 ml tube having a open, threaded end 32 and a closed end 34.

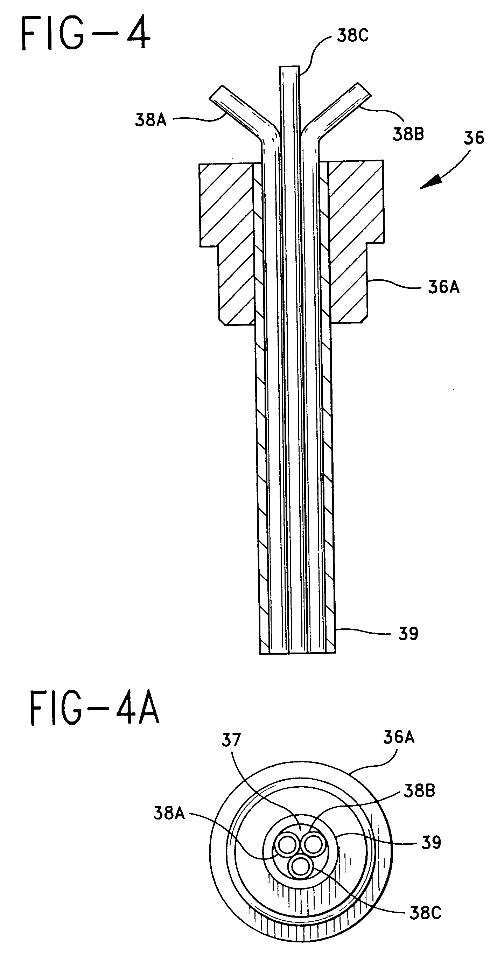

A bulkhead assembly 36 is positioned in the open end 32 of the tube 30, which is illustrated in greater detail in Figures 4 and 4A. The bulkhead assembly 36 includes a bulkhead fitting 36A having at least one hole 37, with three interconnection members 38A, 38B and 38C extending through the hole 37. In the preferred embodiment, the bulkhead assembly remains stationary by being, for example, affixed to a frame (not shown) with the interconnection members made of, for example, metal tubing having an outer diameter slightly larger than the inner diameter of the tubing 24 so that they can be slipped over the interconnection members. The bulkhead assembly also includes a sleeve 39 of, for example, metal encircling the lower portions of the interconnection members extendable into the tube 30, with the space therein between the interconnection members 38A, 38B and 38C and the sleeve 39 filled with an epoxy material.

A length of tubing 24A connects the source 12 of fluid with the tube 30 through interconnection member 38A, with a supply or sort valve 26 positioned along the length of tubing 24A to control the flow of fluid from the source to the tube 30.

As illustrated in Figures 1 and 2, the filtration assembly 16 generally includes a first or top portion 40 and a second or bottom portion 42 with a filter membrane 44 sandwiched therebetween. The membrane is chose to selectively exclude the objects 13A taking into consideration the desired characteristics, such as size, of the objects to be separated. However, it should be appreciated that the membrane or a combination of membranes could be selected depending upon the desired characteristics of the objects to be separated.

The first portion 40 of the filtration assembly also includes an inlet 46 and an outlet 48, with the inlet 46 interconnected in fluid communication with the tube 30 by a length of tubing 24B through the hole 37 in the bulkhead fitting 36A and with the outlet 48 interconnected in fluid communication with the tube 30 by a length of tubing 24C also through the hole 37. The second portion 42 of the filtration assembly 16 includes an outlet 50 through which fluid is drawn off from the filtration assembly 16 through the filter membrane 44. Situated within each of the portions 40 and 42 is a filtration assembly flow pathway, including an upper pathway 51A and a lower pathway 51B separated by the filter membrane 44.

A pump 52 is provided along the length of tubing 24C in fluid communication therewith for circulating the fluid from the tube 30, through interconnection member 38C, the filtration assembly 16 and back to the tube 30.

Another pump 54 is connected to the outlet 50 of the filtration assembly by a length of tubing 24D to provide a suction force for drawing a portion of the fluid 13 off, through the filter member 44 as effluent to comprise a filtrate removal sub-assembly. In the preferred embodiment, the effluent may be deposited and stored in a collection member 56 as waste. However, it should be appreciated that collection and storage of the effluent may be desired, with the particles or the like separated therefrom being discarded as waste or otherwise used.

A level maintenance/concentration valve 58, e.g., a 3-way valve in the preferred embodiment, is provided along a length of tubing 24D so that when the level maintenance/concentration valve 58 is in a first position (opened), as indicated by solid lines in Figure 1, a suction force is provided through the length of tubing 24D and when the valve is in a second position (closed), as indicated by dashed lines (phantom) in Figure 1, the suction force is diverted to atmosphere air through a length of tubing 24D' and check valve 60. The check valve 60 is provided at the end thereof to prevent aerosoling or misting of filtrate to the atmosphere through the length of tubing 24D'.

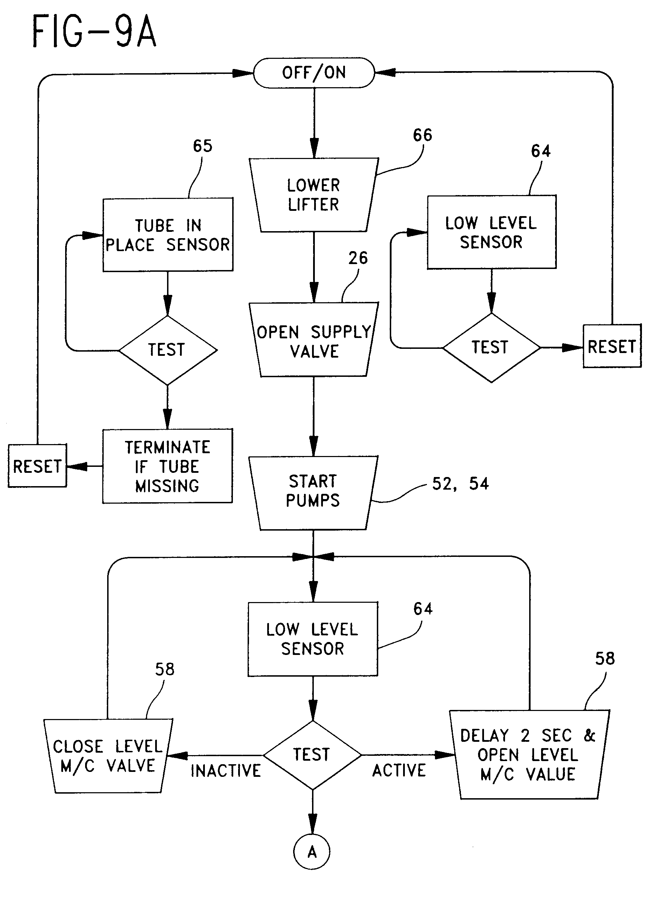

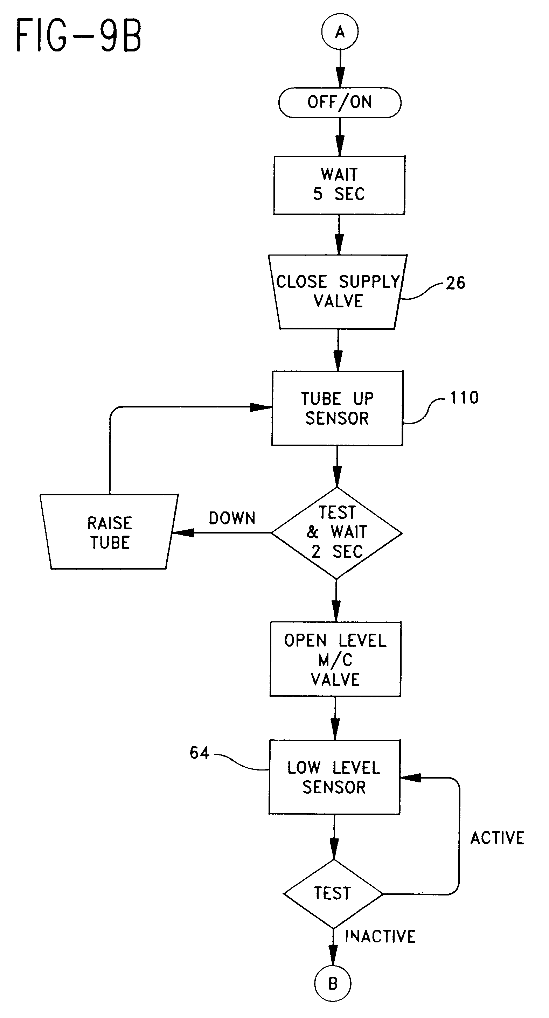

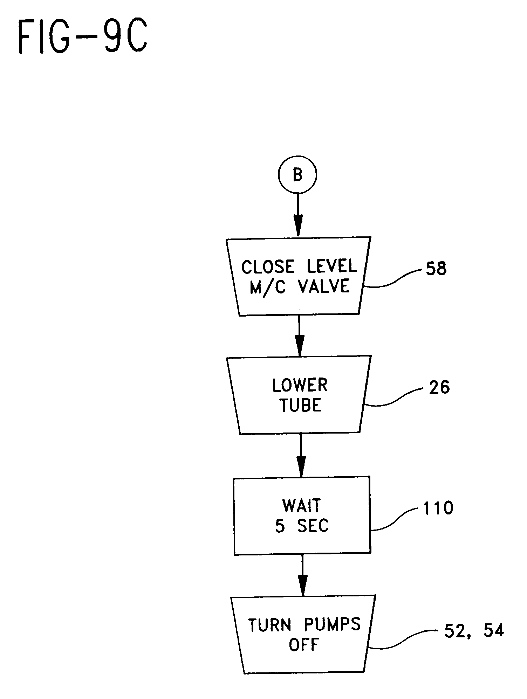

As illustrated in Figure 1, the sensor assembly 20 includes a high level sensor assembly 62, a low level sensor assembly 64, a tube in place sensor 65 and a lifter 66 for raising and lowering the tube 30 according to the system operation disclosed hereinbelow in greater detail in connection with Figures 9A, 9B and 9C.

A pneumatic cylinder 68 is connected to the lifter 16 and in communication with a source 72 of compressed air by a length of tubing 24E so that when compressed air is supplied to the cylinder 68, the lifter 66 and the tube 30 are raised, and when air is released from the cylinder 68, the lifter 66 and the tube 30 are lowered. A 3-way valve 70 is positioned along the length of tubing 24E to control the supply of compressed air thereto, with a length of tubing 24E' provided through which the compressed air is released from the cylinder. In addition, flow restrictor 74 can be provided at the end of the length of tubing 24E' to control the flow of compressed air into and out of the cylinder 68. It should be appreciated that the particular arrangement and force provided for raising and lowering the tube 30 are not essential to the present invention and may include hydraulic cylinders, electric solenoids, a motorized lifter and the like.

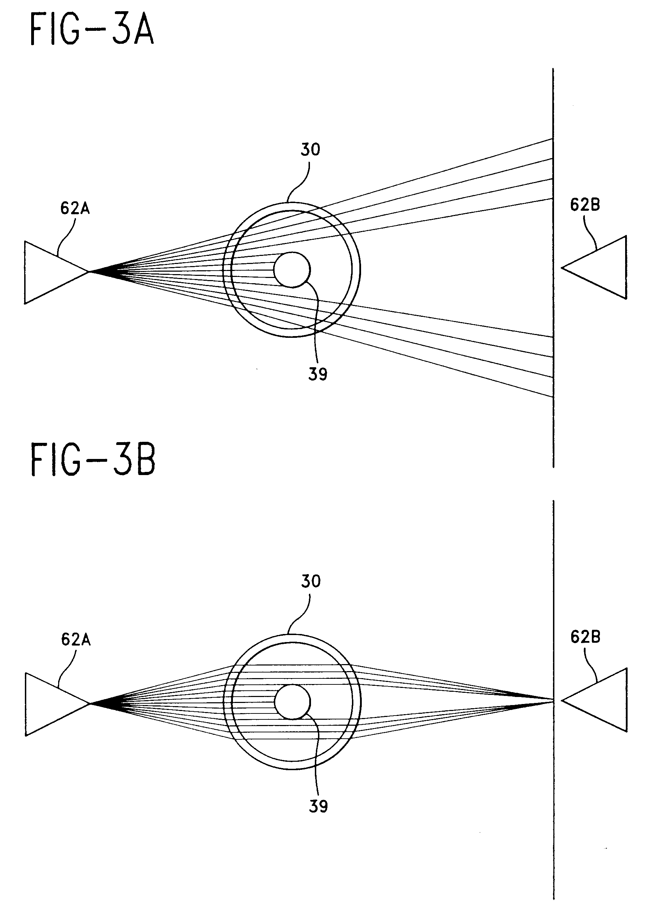

As illustrated in Figure 1, and in greater detail in Figures 3A and 3B, the high and low level sensor assemblies 62 and 64 each includes a light source 62A, 64A and a photo detector 62B, 64B, respectively. In this way, as illustrated in Figure 3A, light emitted from the light source passes through tube 30 and is diffused and further blocked by the sleeve 39 of the bulkhead assembly 36 from activating the photo detector. However, as illustrated in Figure 3B, light emitted from light source when fluid is present in the pathway is refracted and focused by the fluid to activate the photo detector.

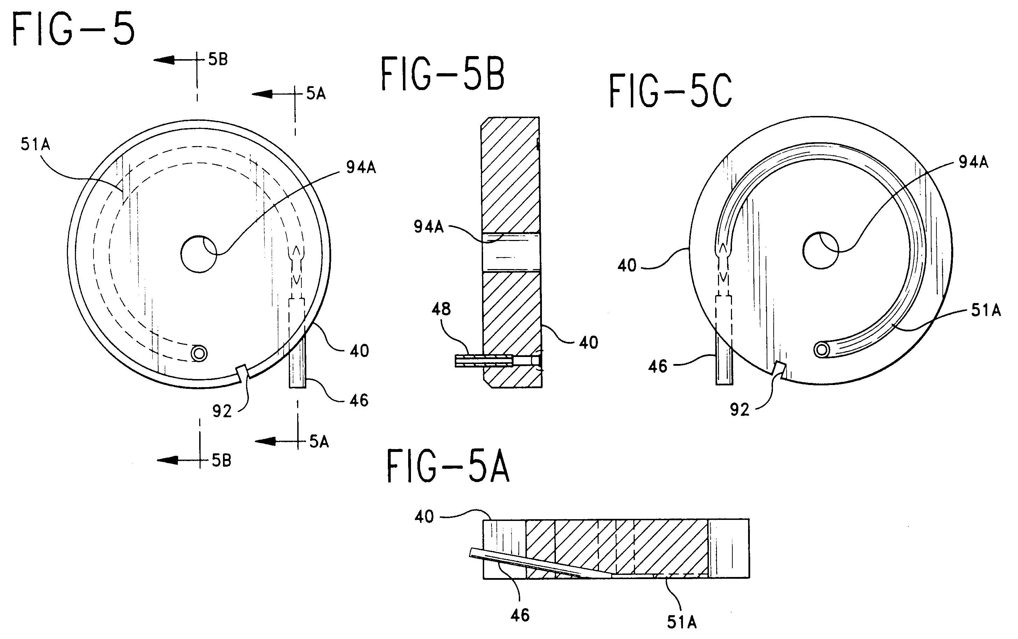

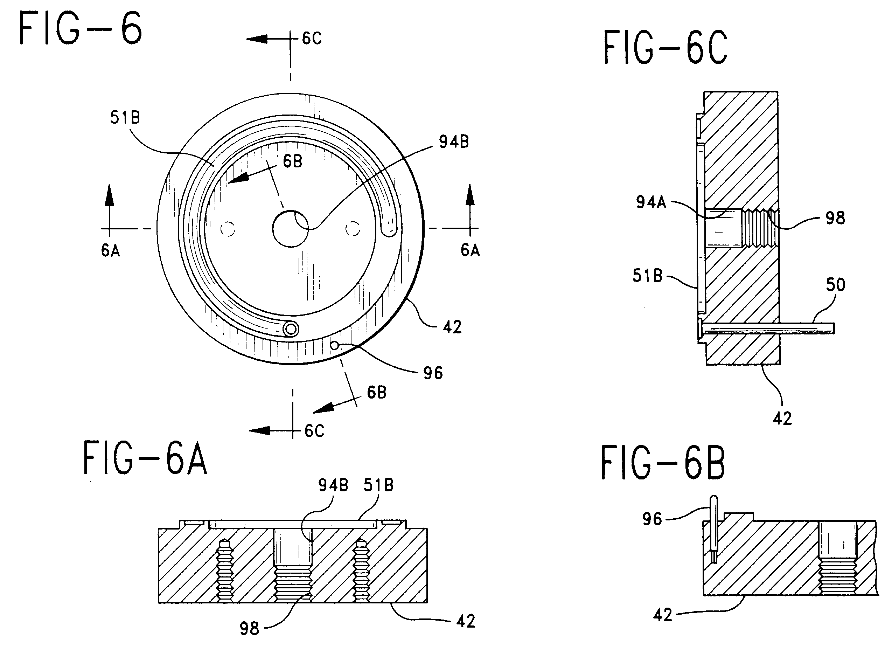

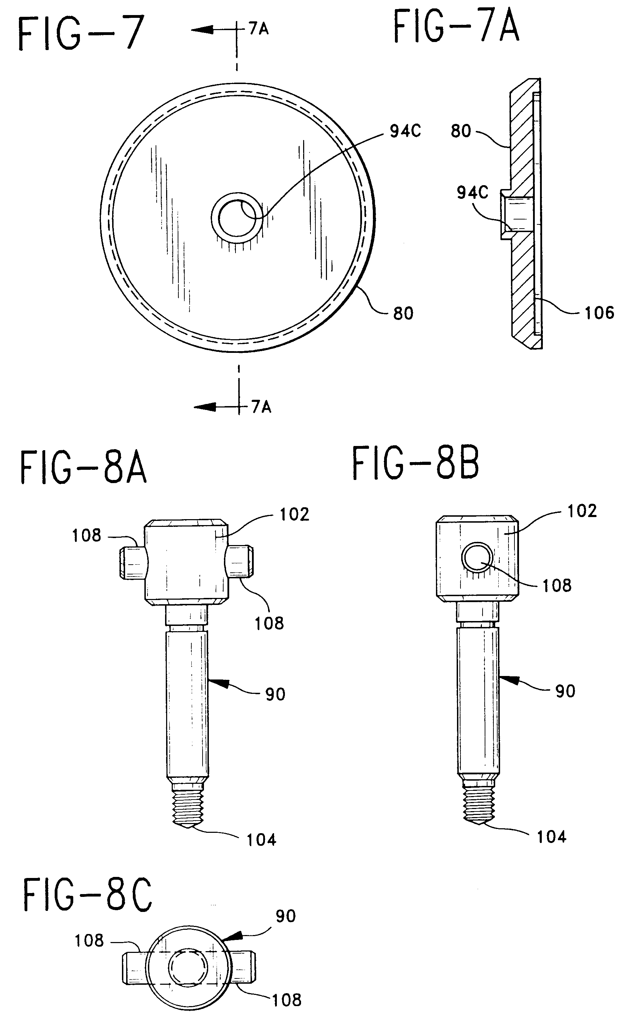

The various components of the filtration assembly 16 of the preferred embodiment are illustrated in Figures 1 and 2, and in greater detail in Figures 5-8. Specifically, Figures 5, 5A, 5B and 5C illustrate the first portion 40 and Figures 6, 6A, 6B and 6C illustrate the second portion 42, both of which are, in the preferred embodiment circular in shape. In addition, Figures 7 and 7A illustrate a clamping washer 80 and Figures 8A, 8B and 8C illustrate a screw for holding the first and second portions 40 and 42 of the filtration assembly 16 together.

As illustrated in Figures 5, 5A, 5B and 5C, the upper pathway 51A is circular in shape and the inlet 46 forms an acute angle with the pathway, which in the preferred embodiment is approximately 10° to provide a turbulent free flow of the fluid and objects suspended therein through the first portion 40 of the filtration assembly, and the outlet 48 forms a right angle with the pathway. As illustrated in Figures 5 and 5C, a slot 92 is provided on the first portion 40 of the filtration assembly 16 to insure proper alignment with the second portion 42. In addition, a central bore 94A in provided in the middle of the first portion of the filtration assembly.

As illustrated in Figures 6, 6A, 6B and 6C, the lower pathway 51B is also circular in shape and outlet 50 forms a right angle with the pathway. As illustrated in Figures 6 and 6B, a pin 96 extends from the second portion 42 and fits in the slot 92 to insure proper alignment between the two portions of the filtration assembly 16. In addition, a central bore 94B in also provided in the middle of the second portion of the filtration assembly, with a threaded portion 98 at the end thereof.

As illustrated in Figures 7 and 7A, the clamping washer 80 also has a central bore 94C. As illustrated in Figures 8A, 8B and 8C, the screw 90 is provided with a head 102 and a threaded distal end 104, with the head in the preferred embodiment being provided with extending portions 108 for engagement with a torque wrench. Accordingly, to assemble the filtration assembly 16, the screw 90 is simply passed through the clamping washer 80, the first portion 40 and the second portion 42 along the bores 94A, 94B, 94C and threadingly engaged with the threaded portion 98 of the second portion to tighten the filtration assembly together with the filter membrane 44 therebetween. The clamping washer 80, as illustrated in Figure 7A, is provided with a relief 106 so that the clamping force resulting form tightening the screw 90 is exerted along the outer peripheral of the filtration assembly.

The materials and components used for constructing the various assemblies are also not essential to the present invention and may be made from a variety of materials and selected from a number of sources as is well known to those skilled in the art. Normally, manufacturers of the present apparatus will select the various materials and components, based upon price, availability and application, i.e., whether the apparatus will be integrated into a flow cytometer or function as a stand alone item.

For example, the various lengths of tubing 24 may be made from a plastic material having the properties of biocompatibility, flexibility, and durability such as polymers, including polyvinylchloride, polyolefin, silicon etc.

### Operation and Use

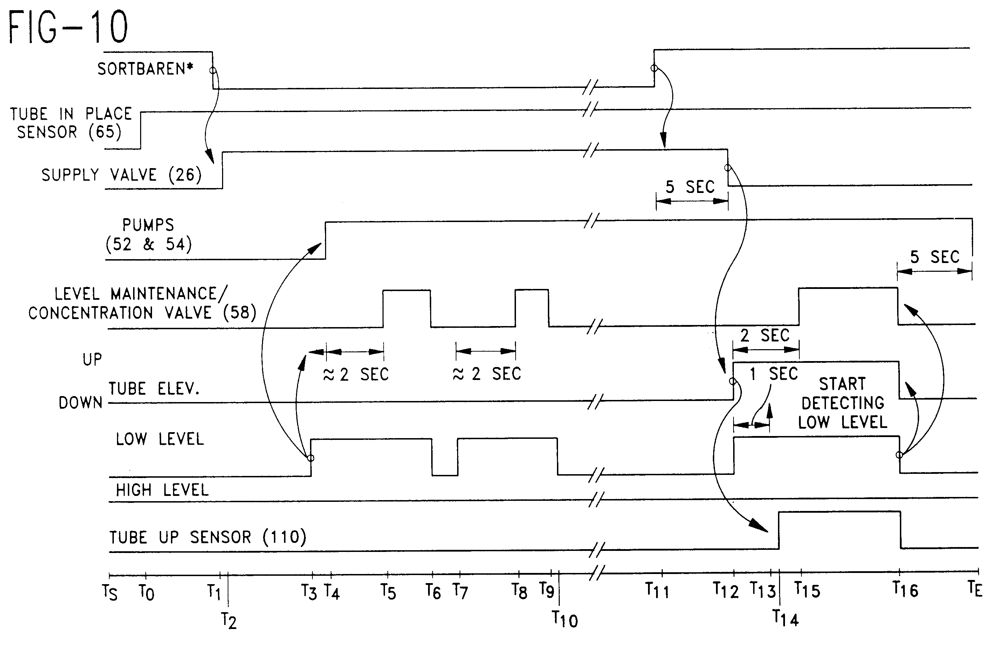

The operation of the system of the present invention in connection with the apparatus 10 illustrated in Figures 1 and 2 will now be explained with reference to the flow diagram shown in Figures 9A, 9B and 9C and the timing diagram shown in Figure 10.

Usually, the fluid 13 with the objects 13A to be concentrated suspended therein is supplied to the collection member 14. Accordingly, the concentration phase of the system is initiated at time T_{S}, with the tube positioning sub-assembly 28 determining if the tube 16 is in place (T₀) and the supply valve 26 being opened (T₂) to provide fluid to the system. As illustrated in Figure 10, the system may be connected to a cytometer in which case it would be initiated to begin operation at time T₁ before the supply valve 26 is opened.

A portion of this fluid 13 is preferably continuously circulated through the filtration assembly 16 by the lengths of tubing 24B and 24C tangentially along the filter membrane 44 with sufficient force to prevent the objects 13A from being lodged against the filter membrane 44. In this way, as the fluid passes through the filtration assembly 16 along the filter membrane, a portion of the fluid is drawn through the membrane by the negative pressure so that less than the initial portion of the fluid is returned to the collection member 14 with a higher concentration of objects suspended therein.

The high and low level sensor assemblies 62 and 64 upon initiation of the system begin sensing the level of the fluid in the tube (T₃). The pumps 52 and 54 are then activated (T₄) to continuously circulate the fluid 13 through the filtration assembly 16. Thereafter, if the level of the fluid drops and reaches a point to deactivate the low level sensor 64, the level maintenance/concentration valve 58 is closed to vent the suction to the atmosphere (T₅). In response thereto, the level of the fluid rises and the level maintenance/concentration valve 58 is again closed (T₆).

The level of the fluid in the tube 30 of the collection member 14 is continuously monitored to prevent the tube from overflowing and to prevent the objects 13A suspended in the fluid 13 from becoming too concentrated, i.e., not enough fluid to suspend them therein and maintain circulation along the flow path without causing damage to the objects. A delay of two seconds, is provided to the cycle to maintain continuous flow of fluid there through (T₇-T₁₀) within an acceptable range and to extend the life of the valves. In addition, in this way, additional fresh fluid 13 can be continuously supplied to the collection member 14 while a portion of it is cycled through the filtration assembly 16.

When the supply of fresh fluid is exhausted (T₁₁) and/or the system is turned off, after a delay of five seconds, the supply valve 26 closes (T₁₂) to draw off any remaining fluid from the source 72 and after a delay of one second (T₁₃), a tube up sensor 110 detects whether the tube is in the up position (T₁₄). If not, the tube is raised.

After a delay of two seconds from the time the tube is raised, the level maintenance/concentration valve 58 is opened to complete concentration of the remaining fluid (T₁₅).

Prior to and during this time period, the low level sensor 64 is activated. However, upon deactivation of the sensor 64, i.e., the level falls below the low level sensor 64 (T₁₆), the level maintenance/concentration valve 58 is closed, the tube is lowered below the end of the sleeve 39 to scavenge or recover any fluid 13, and the objects 13A suspended therein circulating through the system, by permitting them to drain out of the system into the tube 30. After a five second delay during which the system is drained, the pumps 52 and 54 are turned off.

While a preferred embodiment of the present invention has been described so as to enable one skilled in the art to practice the device of the present invention, it is to be understood that variations and modifications may be employed without departing from the concept and intent of the present invention as defined in the following claims. The preceding description is intended to be exemplary and should not be used to limit the scope of the invention. The scope of the invention should be determined only by reference to the following claims.

## Claims

1. An apparatus for separating objects such as cells, particles and the like from at least a portion of a fluid in which they are contained or otherwise suspended, comprising:
source means for supplying a fluid with said objects to be separated suspended therein;
collection means for retaining at least partially diluted objects;
filtration means for filtering at least a portion of said objects from said fluid;
circulating means for circulating said fluid from said collection means through said filtration means while at least a portion of said fluid may be drawn off to concentrate said objects in a remaining portion of said fluid, while permitting the objects suspended in the remaining portion of said fluid to continue to be circulated through the apparatus; and
fluid level control means for monitoring and controlling the level of fluid in said collection means.

2. The apparatus defined in claim 1, wherein said fluid level control means is adapted to include said sensing means for detecting when said fluid approaches a high level point and for detecting when said fluid approaches a low level point and is adapted to include a lifting means for raising and lowering said collection means.

3. The apparatus defined in claim 2, wherein said lifting means includes a lifter valve, a source of compressed air and a cylinder connected to said lifer, with said cylinder in communication with said source of compressed air and said fluid level control means is also adapted to lower said collection means to drain off any remaining fluid from said flow path upon completion of a concentration phase.

4. The apparatus defined in claim 1, wherein said filtration means is adapted to include a filter membrane positioned along a flow path through which said portion of fluid is drawn off and said circulating means is adapted to include a circulating pump for circulating said fluid from said collection means through said filter assembly and back to said collection means.

5. The apparatus defined in claim 4, wherein said filtration means is adapted to include filtrate removal means for drawing off said portion of said fluid through said filter membrane, with said filtrate removal means including at least one pump and a valve so that when said valve is in a first position said portion of fluid is drawn through said filter membrane.

6. A method for separating objects such as cells, particles and the like from at least a portion of a fluid in which such objects are suspended, comprising the steps of:
providing said fluid with said objects suspended therein to a collection member;
circulating said fluid from said collection member along a flow path through a filtration assembly and returning at least a remaining portion of said fluid to said collection member with a higher concentration of objects suspended therein;
drawing off a portion of said fluid from said filtration assembly as said fluid circulates therethrough, while permitting the objects suspended in the remaining fluid to continue to be circulated along said flow path; and
monitoring and controlling the level of said fluid in said collection member.

7. The method defined in claim 6, further comprising the step of passing said fluid through said filtration assembly tangentially to a first side of a filter membrane with sufficient force to prevent said objects from being lodged against said first side of said filter membrane while permitting said portion of said fluid to pass through said filter membrane and be drawn off from the remaining portion of fluid circulating along said flow path through said circulating means.

8. The method defined in claim 7, further comprising the step of providing a negative pressure difference to a second side of said filter assembly so that said portion of said fluid is drawn through the filter membrane to a holding member.

9. The method defined in claim 8, wherein said step of passing said fluid through said filtration assembly tangentially to a first side of a filter membrane includes initially directing said fluid towards said filter membrane at an acute angle.

10. The method defined in claim 6, further comprising the steps of raising said collection member to further concentrate the objects in said fluid and thereafter lowering said collection member to drain off any remaining fluid from said flow path and sensing whether the tube is in place, and resetting the system if the sensor is activated.
